(19) Europäisches Patentamt
European Patent Office
Office européen des brevets

(11) Publication number: 0 319 167 A1

# (12) EUROPEAN PATENT APPLICATION

(21) Application number: 88310821.9

(22) Date of filing: 16.11.88

(51) Int. Cl.[4]: C07D 239/34 , C09K 19/34

(30) Priority: 26.11.87 JP 298754/87

(43) Date of publication of application: 07.06.89 Bulletin 89/23

(84) Designated Contracting States: CH DE FR GB LI

(71) Applicant: Chisso Corporation
6-32, Nakanoshima 3-chome Kita-ku
Osaka-shi Osaka-fu(JP)

(72) Inventor: Ohno, Kouji
10-3, Otsutomo-cho Kanazawa-ku
Yokohama-shi Kanagawa-ken(JP)
Inventor: Ushioda, Makoto
12-16, Kannon 1-chome Kawasaki-ku
Kawasaki-shi Kanagawa-ken(JP)
Inventor: Miyazawa, Kazutoshi
12-14, Mutsuura 2-chome Kanazawa-ku
Yokohama-shi Kanagawa-ken(JP)
Inventor: Inoue, Hiromichi
10-2, Otsutomo-cho Kanazawa-ku
Yokohama-shi Kanagawa-ken(JP)
Inventor: Saito, Shinichi
10-1, Otsutomo-cho Kanazawa-ku
Yokohama-shi Kanagawa-ken(JP)

(74) Representative: Lamb, John Baxter et al
MARKS & CLERK 57/60 Lincoln's Inn Fields
London WC2A 3LS(GB)

## (54) 2-Phenyl-5-alkanoyloxypyrimidines.

(57) Non-optically active, liquid crystalline compounds capable of acting as a base material for ferroelectric liquid crystal compositions, have the formula:

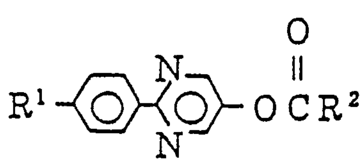

(I)

wherein $R^1$ is a $C_1$-$C_{20}$ alkyl or alkoxy group and $R^2$ is a $C_1$-$C_{20}$ alkyl group.

EP 0 319 167 A1

## 2-Phenyl-5-alkanoyloxypyrimidines

This invention relates to a novel liquid crystalline compound useful for display elements and a chiral smectic liquid crystal composition comprising the above-mentioned liquid crystalline compound and optically active compound(s).

At present, TN (Twisted Nematic) display mode has been most broadly employed, but it is inferior in the aspect of the response rate to emissive display elements such as those of electroluminescence display, plasma display, etc. and hence various attempts of improvement in this respect have been made, and nevertheless a possibility of the improvement to a large extent seems to remain so much. Thus, various attempts at liquid crystal display devices based on a different principle in place of that of TN mode display elements, and among these, there is a display method utilizing ferroelectric liquid crystals (N.A. Clark et al, Applied Phys. lett., 36, 899 (1980)). This mode utilizes chiral smectic C phase (hereinafter abbreviated to SC* phase) of ferroelectric liquid crystals or smectic phase exhibiting ferroelectricity, and the temperature range exhibiting these phases are preferred to be in the vicinity of room temperature.

It has been known that these chiral smectic liquid crystal materials may be obtained by blending a plurality of single compounds exhibiting chiral smectic phase by themselves, but the materials may also be obtained by adding at least one member of optically active compounds, preferably optically active liquid crystal compounds, more preferably chiral smectic liquid crystal compounds to achiral smectic liquid crystals (liquid crystal compounds exhibiting smectic C (SC) phase or the like). Various substances exhibiting SC phase or the like have been known, but as to whether chiral smectic liquid crystal materials obtained by adding optically active compounds to the above-mentioned substances exhibit superior performances, ultimate evaluation has not yet been obtained. It is because liquid crystal display utilizing ferroelectricity has not yet been technically completed. Thus, in the present status, it is necessary to find novel liquid crystal materials and carry out various tests thereon.

### SUMMARY OF THE INVENTION

The main object of the present invention is to provide a novel, non-optically active, liquid crystalline compound suitable to the above-mentioned use application, that is, capable of constituting the base substance of ferroelectric liquid crystal compositions. In addition, the liquid crystalline compound referred to herein also includes compounds which, even when they exhibit no liquid crystal state by themselves, have a chemical structure similar to those of liquid crystal compounds and can constitute a component of liquid crystal compositions.

Liquid crystalline compound herein described can include a compound which has a chemical structure similar to a liquid crystal compound and is capable of being a component of liquid crystal composition, even if it exhibits no liquid crystal properties.

The present invention resides in:

A compound expressed by the formula

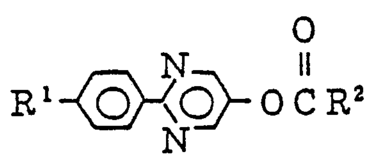

(I)

wherein $R^1$ represents an alkyl group or an alkoxy group each of 1 to 20 carbon atoms and $R^2$ represents an alkyl group of 1 to 20 carbon atoms;

a chiral smectic composition comprising at least one member of the above compound and at least one optically active compound; and

a light switching element using the chiral smectic composition.

### DETAILED DESCRIPTION OF PREFERRED EMBODIMENTS

The carbon number of the alkyl group or alkoxy group of $R^1$ in the formula (I) is preferably 4 to 16, more preferably 6 to 12 and that of the alkyl group of $R^2$ therein is preferably 5 to 15, more preferably 6 to 12.

Concrete examples of the compound of the formula (I) are shown below and the phase transition points of representative examples of the compound of the formula (I) are shown in Table 1. 5-Hexanoyloxy-2-(4-hexylphyenyl)pyrimidine,
5-Heptanoyloxy-2-(4-hexylphenyl)pyrimidine,
2-(4-Hexylphenyl)-5-octanoyloxypyrimidine,
2-(4-Hexylphenyl)-5-nonanoyloxypyrimidine,
5-Decanoyloxy-2-(4-hexylphenyl)pyrimidine,
2-(4-Hexylphenyl)-5-undecanoyloxypyrimidine,
5-Dodecanoyloxy-2-(4-hexylphenyl)pyrimidine,
2-(4-Hexylphenyl)-5-tridecanoyloxypyrimidine,
5-Hexanoyloxy-2-(4-heptylphenyl)pyrimidine,
5-Heptanoyloxy-2-(4-heptylphenyl)pyrimidine

(Sample No. 1)

2-(4-Heptylphenyl)-5-octanoyloxypyrimidine,
2-(4-Heptylphenyl)-5-nonanoyloxypyrimidine,
5-Decanoyloxy-2-(4-heptylphenyl)pyrimidine,
2-(4-Heptylphenyl)-5-undecanoyloxypyrimidine,
5-Dodecanoyloxy-2-(4-heptylphenyl)pyrimidine,
2-(4-Heptylphenyl)-5-tridecanoyloxypyrimidine,
5-Hexanoyloxy-2-(4-octylphenyl)pyrimidine,
5-Heptanoyloxy-2-(4-octylphenyl)pyrimidine,
5-Octanoyloxy-2-(4-octyl phenyl)pyrimidine

(Sample No. 2)

5-Nonanoyloxy-2-(4-octylphenyl) pyrimidine,
5-Decanoyloxy-2-(4-octylphenyl)pyrimidine,
2-(4-Octylphenyl)-5-undecanoyloxypyrimidine,
5-Dodecanoyloxy-2-(4-octylphenyl)pyrimidine,
2-(4-Octylphenyl)-5-tridecanoyloxypyrimidine,
5-Hexanoyloxy-2-(4-nonylphenyl)pyrimidine,
5-Heptanoyloxy-2-(4-nonylphenyl)pyrimidine,
2-(4-Nonyllphenyl)-5-octanoyloxypyrimidine,
5-Nonanoyloxy-2-(4-nonylphenyl)pyrimidine,
5-Decanoyloxy-2-(4-nonylphenyl)pyrimidine,
2-(4-Nonylphenyl)-5-undecanoyloxypyrimidine,
5-Dodecanoyloxy-2-(4-nonylphenyl)pyrimidine,
2-(4-Nonylphenyl)-5-tridecanoyloxypyrimidine,
2-(4-Decylphenyl)-5-hexanoyloxypyrimidine,
2-(4-Decylphenyl)-5-heptanoyloxypyrimidine,
2-(4-Decyllphenyl)-5-octanoyloxypyrimidine,
2-(4-Decylphenyl)-5-nonanoyloxypyrimidine,
5-Decanoyloxy-2-(4-decylphenyl)pyrimidine,
2-(4-Decylphenyl)-5-undecanoyloxypyrimidine,
2-(4-Decylphenyl)-5-dodecanoyloxypyrimidine,
2-(4-Decylphenyl)-5-tridecanoyloxypyrimidine,
5-Hexanoyloxy-2-(4-undecylphenyl)pyrimidine,
5-Heptanoyloxy-2-(4-undecylphenyl)pyrimidine,
5-Octanoyloxy-2-(4-undecyllphenyl)pyrimidine,
5-Nonanoyloxy-2-(4-undecylphenyl)pyrimidine,

5-Decanoyloxy-2-(4-undecylphenyl)pyrimidine,
5-Undecanoyloxy-2-(4-undecylphenyl)pyrimidine,
5-Dodecanoyloxy-2-(4-undecylphenyl)pyrimidine,
5-Tridecanoyloxy-2-(4-undecylphenyl)pyrimidine,
2-(4-Dodecylphenyl)-5-hexanoyloxypyrimidine,
2-(4-Dodecylphenyl)-5-heptanoyloxypyrimidine,
2-(4-Dodecylphenyl)-5-octanoyloxypyrimidine,
2-(4-Dodecylphenyl)-5-nonanoyloxypyrimidine,
5-Decanoyloxy-2-(4-dodecylphenyl)pyrimidine,
2-(4-Dodecylphenyl)-5-undecanoyloxypyrimidine,
5-Dodecanoyloxy-2-(4-dodecylphenyl)pyrimidine,
2-(4-Dodecylphenyl)-5-tridecanoyloxypyrimidine,
5-Hexanoyloxy-2-(4-hexyloxyphenyl)pyrimidine,
5-Heptanoyloxy-2-(4-hexyloxyphenyl)pyrimidine,
2-(4-Hexyloxyphenyl)-5-octanoyloxypyrimidine

(Sample No. 3)

2-(4-Hexyloxyphenyl)-5-nonanoyloxypyrimidine,
5-Decanoyloxy-2-(4-hexyloxyphenyl)pyrimidine,
2-(4-Hexyloxyphenyl)-5-undecanoyloxypyrimidine,
5-Dodecanoyloxy-2-(4-hexyloxyphenyl)pyrimidine,
2-(4-Hexyloxyphenyl)-5-tridecanoyloxypyrimidine,
5-Hexanoyloxy-2-(4-heptyloxyphenyl)pyrimidine,
5-Heptanoyloxy-2-(4-heptyloxyphenyl)pyrimidine

(Sample No. 4)

2-(4-Heptyloxyphenyl)-5-octanoyloxypyrimidine,
2-(4-Heptyloxyphenyl)-5-nonanoyloxypyrimidine,
5-Decanoyloxy-2-(4-heptyloxyphenyl)pyrimidine

(Sample No. 5)

2-(4-Heptyloxyphenyl)-5-undecanoyloxypyrimidine,
5-Dodecanoyloxy-2-(4-heptyloxyphenyl)pyrimidine
2-(4-Heptyloxyphenyl)-5-tridecanoyloxypyrimidine,
5-Hexanoyloxy-2-(4-octyloxyphenyl)pyrimidine,
5-Heptanoyloxy-2-(4-octyloxyphenyl)pyrimidine

(Sample No. 6)

5-Octanoyloxy-2-(4-octyloxyphenyl)pyrimidine,
5-Nonanoyloxy-2-(4-octyloxyphenyl)pyrimidine,
5-Decanoyloxy-2-(4-octyloxyphenyl)pyrimidine,
2-(4-Octyloxyphenyl)-5-undecanoyloxypyrimidine,
5-Dodecanoyloxy-2-(4-octyloxyphenyl)pyrimidine,
2-(4-Octyloxyphenyl)-5-tridecanoyloxypyrimidine,
5-Hexanoyloxy-2-(4-nonyloxyphenyl)pyrimidine,
5-Heptanoyloxy-2-(4-nonyloxyphenyl)pyrimidine,
2-(4-Nonyloxyphenyl)-5-octanoyloxypyrimidine

(Sample No. 7)

5-Nonanoyloxy-2-(4-nonyloxyphenyl)pyrimidine,
5-Decanoyloxy-2-(4-nonyloxyphenyl)pyrimidine,
2-(4-Nonyloxyphenyl)-5-undecanoyloxypyrimidine,
5-Dodecanoyloxy-2-(4-nonyloxyphenyl)pyrimidine,
2-(4-Nonylphenyl)-5-tridecanoyloxypyrimidine,
2-(4-Decyloxyphenyl)-5-hexanoyloxypyrimidine,
2-(4-Decyloxyphenyl)-5-heptanoyloxypyrimidine,
2-(4-Decyloxyphenyl)-5-octanoyloxypyrimidine,
2-(4-Decyloxyphenyl)-5-nonanoyloxypyrimidine

(Sample No. 8)

5-Decanoyloxy-2-(4-decyloxyphenyl)pyrimidine,
2-(4-Decyloxyphenyl)-5-undecanoyloxypyrimidine,
2-(4-Decyloxyphenyl)-5-dodecanoyloxypyrimidine,
2-(4-Decyloxyphenyl)-5-tridecanoyloxypyrimidine,
5-Hexanoyloxy-2-(4-undecyloxyphenyl)pyrimidine,
5-Heptanoyloxy-2-(4-undecyloxyphenyl)pyrimidine,
5-Octanoyloxy-2-(4-undecyloxyphenyl)pyrimidine,
5-Nonanoyloxy-2-(4-undecyloxyphenyl)pyrimidine,
5-Decanoyloxy-2-(4-undecyloxyphenyl)pyrimidine,
5-Undecanoyloxy-2-(4-undecyloxyphenyl)pyrimidine,
5-Dodecanoyloxy-2-(4-undecyloxyphenyl)pyrimidine,
5-Tridecanoyloxy-2-(4-undecyloxyphenyl)pyrimidine,
2-(4-Dodecyloxyphenyl)-5-hexanoyloxypyrimidine,
2-(4-Dodecyloxyphenyl)-5-heptanoyloxypyrimidine,
2-(4-Dodecyloxyphenyl)-5-octanoyloxypyrimidine,
2-(4-Dodecyloxyphenyl)-5-nonanoyloxypyrimidine,
5-Decanoyloxy-2-(4-dodecyloxyphenyl)pyrimidine,
2-(4-Dodecyloxyphenyl)-5-undecanoyloxypyrimidine,
5-Dodecanoyloxy-2-(4-dodecyloxyphenyl)pyrimidine,
2-(4-Dodecyloxyphenyl)-5-tridecanoyloxypyrimidine

Table 1

| Sample No. | In formula (I) | | Phase transition temperature (°C) | | | | | Note |
|---|---|---|---|---|---|---|---|---|
| | $R^1$ | $R^2$ | C | SC | SA | N | I | |
| 1 | $C_7H_{15}$- | -$C_6H_{13}$ | •79.0 | - | - | - | • | |
| 2 | $C_8H_{17}$- | -$C_7H_{15}$ | •79.4 | - | (•74.4) | - | • | Example2 |
| 3 | $C_8H_{13}O$- | -$C_7H_{15}$ | •75.0 | (•74.4) | - | •91.3 | • | |
| 4 | $C_7H_{15}O$- | -$C_6H_{13}$ | •64.9 | •66.2 | - | •85.8 | • | |
| 5 | $C_7H_{15}O$- | -$C_9H_{19}$ | •74.8 | •96.5 | - | - | • | |
| 6 | $C_8H_{17}O$- | -$C_6H_{13}$ | •63.4 | •69.7 | - | •89.7 | • | Example 1 |
| 7 | $C_9H_{19}O$- | -$C_7H_{15}$ | •66.2 | •84.7 | - | •93.5 | • | |
| 8 | $C_{10}H_{21}O$- | -$C_8H_{17}$ | •62.3 | •93.6 | - | •95.3 | • | |

Since the compound of the formula (I) of the present invention mostly exhibits SC phase, a composition consisting of at least one member thereof exhibits SC phase within a broad temperature range. Further, since the compound of the formula (I) of the present invention exhibiting no SC phase also has a molecular structure similar to those of liquid crystals, it is preferably usable as a component constituting SC phase-exhibiting compositions (hereinafter abbreviated to SC composition).

Examples of compounds preferably used together with the compound of the present invention at the time of constituting SC composition are listed as follows.

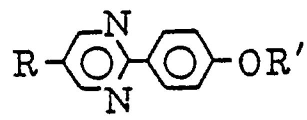

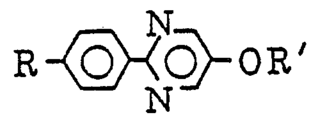

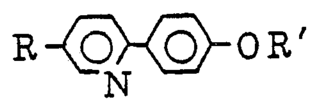

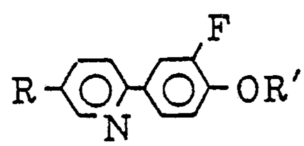

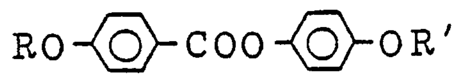

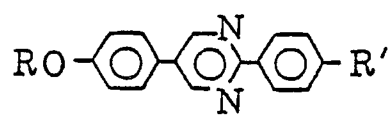

In the above formulas, R and R′ each independently represent an alkyl group.

When a SC composition containing at least one member of the compound of the present invention is blended with other optically active compounds, preferably optically active substances exhibiting chiral smectic C phase, it is possible to obtain a composition exhibiting chiral smectic C phase.

Further, even when optically active substances exhibit no chiral smectic C phase by themselves, if the substances are blended with a SC composition containing at least one member of the compound of the formula (I) of the present invention, a composition exhibiting chiral smectic C phase is obtained.

Examples of such optically compounds are as follows:

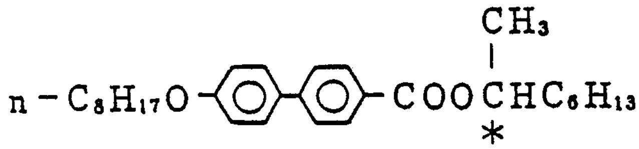

(Japanese patent application laid-open No. Sho 59-118744/1984)

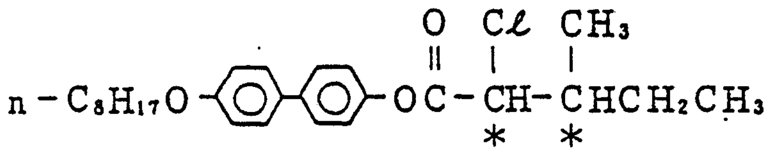

(Japanese patent application laid-open No. Sho 60-218358/1985)

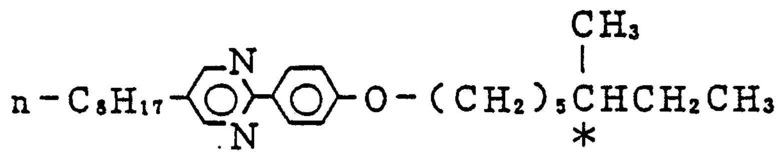

(Japanese patent application laid-open No. Sho 60-260564/1985)

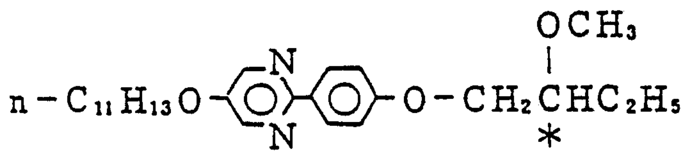

(Preprints for 12th Japan Liquid Crystal Symposium, 2F13)

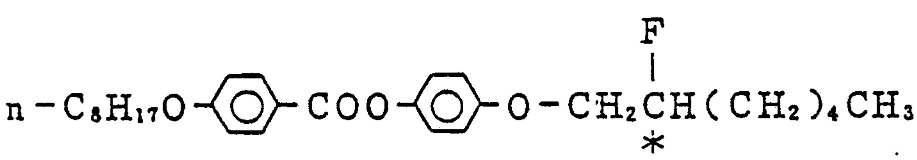

(Preprints for 12th Japan Liquid Crystal Symposium, 2F 11)

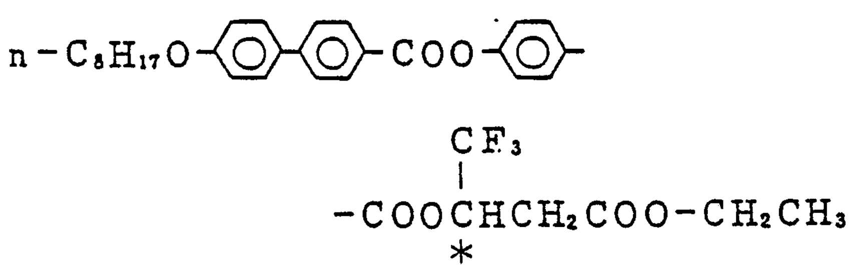

(Chemistry Express Vol. 2, No. 1. pp 53 ~56 (1987))

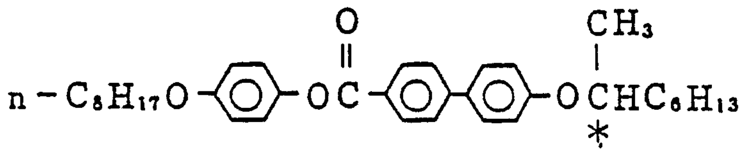

(Japanese patent application laid-open No. Sho 61-43/1986)

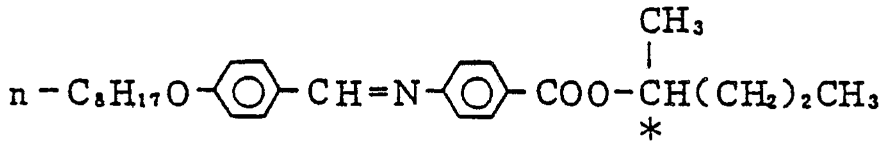

( Ferroelectrics 58, 21 ( 1984 ) )

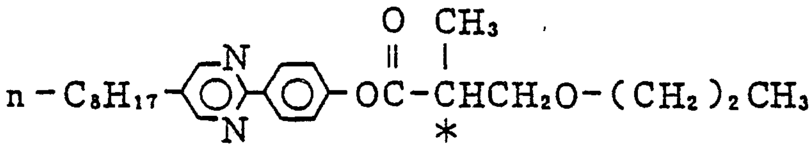

(Japanese patent application laid-open No. Sho 61-44845/1986)

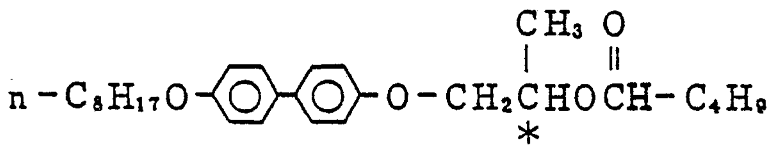

(Japanese patent application No. Sho 61-133269/1986)

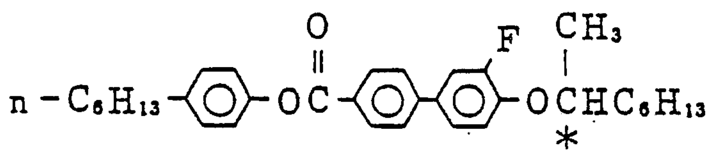

(Japanese patent application No. Sho 61-210056/1986)

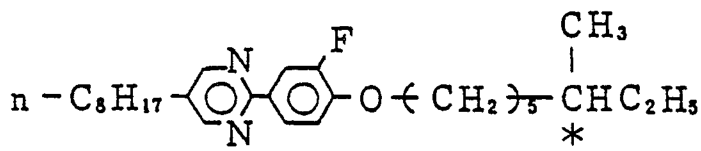

(Japanese patent application No. Sho 61-10578/1986)

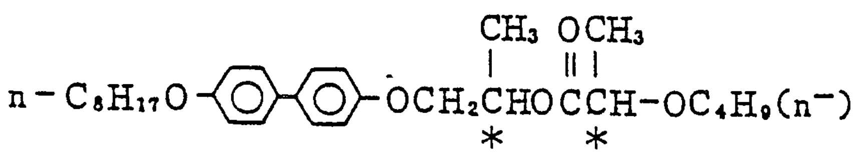

(Japanese patent application No. Sho 62-49796/1987)

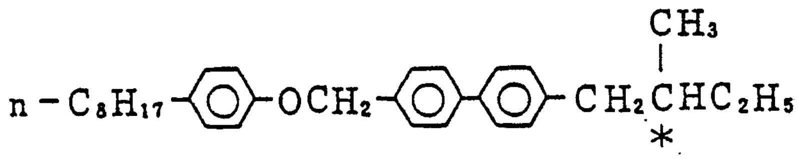

(Japanese patent application laid-open No. Sho 61-63633/1986)

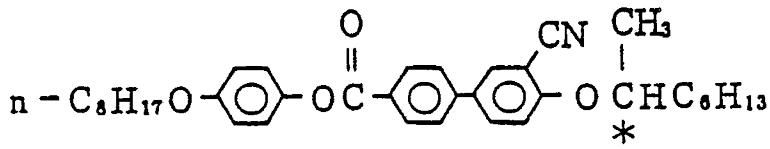

(Japanese patent application No. Sho 61-192516/1986)

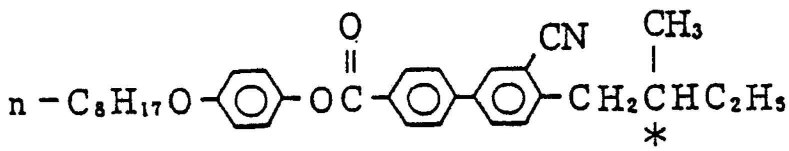

(Japanese patent application No. Sho 62-67097/1987)

The compound of the formula (I) of the present invention may be prepared for example through the following route:

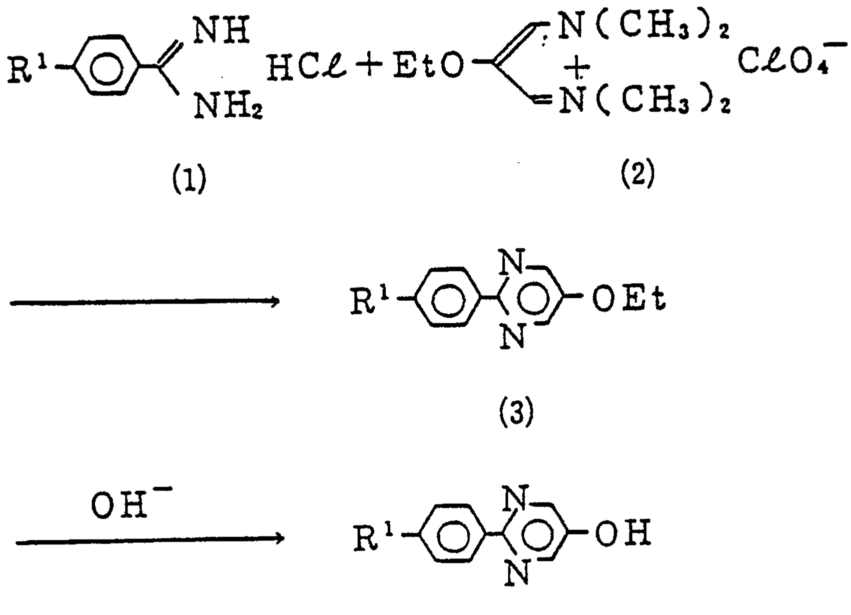


(1) (2)

(3)

(4)

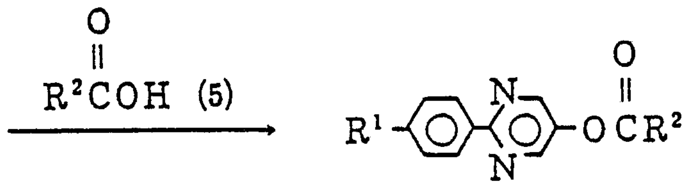


(6)

Namely, a 4-alkylbenzamidine hydrochloride or a 4-alkoxybenzamidine hydrochloride (1) is reacted with 1,3-bis(dimethylamino)-2-ethoxytrimethiunium perchlorate (2) according to a method disclosed in a literature (Collection Czechoslov. Chem. Commun., 38 (1973), 1168) to obtain a compound (3), which is then heated under basic condition to obtain a compound (4), which is esterified to obtain a compound of the formula (I) of the present invention.

In the case of $R^1$ is an alkyloxy group, the compound of the formula (I) may also be prepared through the following route:

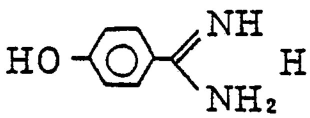

+

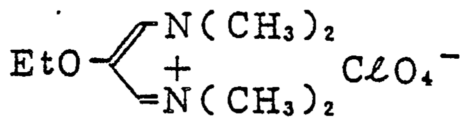

(6) (2)

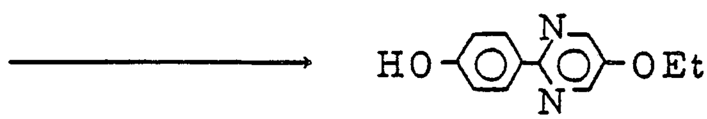

(7)

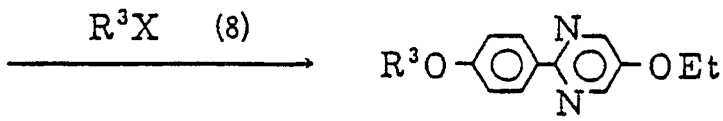


(9)

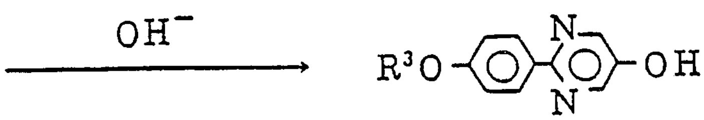


(10)

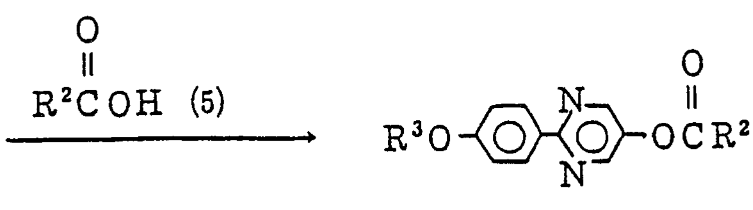


(11)

In the above formulas, $R^3$ represents an alkyl group and X represents a leaving group such as chlorine atom, bromine atom, iodine atom, p-toluenesulfonyloxy group, benzenesulfonyloxy group, methanesulfonyloxy group, etc.

Namely, a 4-hydroxybenzamidine hydrochloride (6) is reacted with a compound (2) to obtain a compound (7), which is reacted with a compound (8) to obtain a compound (9), which is heated under basic condition to obtain a compound (10), which is esterified to obtain a compound (11) which corresponds to a compound of the formula (I) of the present invention wherein $R^1$ represents an alkyloxy group.

The compound of the present invention will be described in more detail by way of Examples.

Example 1

Preparation of 5-heptanoyloxy-2-(4-octyloxyphenyl)pyrimidine

(a compound of the formula (I) wherein $R^1$ represents $C_8H_{17}O$- and $R^2$ represents $-C_6H_{13}$)

(i) Preparation of 5-ethoxy-2-(4-hydroxyphenyl)pyrimidine

To a solution of sodium methylate (194.6 g) in methanol (2,000 mℓ) were added 4-hydroxybenzamidine hydrochloride (200 g) and 1,3-bis(dimethylamino)-2-ethoxytrimethinium perchlorate (313.5 g) prepared according to a method described in a literature (Collection Czechoslov. Chem. Commun., 38 (1973), 1168), followed by keeping the mixture under reflux for 6.5 hours, allowing the resulting material to cool down, adding water until the system became uniform, further adding acetic acid until the mixture was acidified and filtering off deposited crystals to obtain 5-ethoxy-2-(4-hydroxyphenyl)pyrimidine (205 g) (m.p.: 201.5 - 202.3 °C).

(ii) Preparation of 5-ethoxy-2-(4-octyloxyphenyl) pyrimidine

5-Ethoxy-2-(4-hydroxyphenyl)pyrimidine (40 g) was dissolved in ethanol (340 mℓ), followed by adding KOH (11.2 g) and heptyl bromide (35.8 g) to the solution, keeping the mixture under reflux for 3 hours, distilling off ethanol (about 300 mℓ), extracting the residue with toluene (500 mℓ), washing the resulting organic layer with 2N-NaOH aqueous solution and further with water until the washing water became neutral, distilling off the solvent and recrystallizing the residue from ethanol to obtain 5-ethoxy-2-(4-octyloxyphenyl)pyrimidine (46.9 g). This product exhibited nematic phase and its phase transition points were as follows:

$$C \xrightarrow{86.3\,^{\circ}C} N \xrightarrow{93.0\,^{\circ}C} I$$

(iii) Preparation of 5-hydroxy-2-(4-octyloxyphenyl)pyrimidine

5-Ethoxy-2-(4-octyloxyphenyl)pyrimidine (48 g) was dissolved in diethylene glycol (300 mℓ), followed by adding NaOH (58.4 g) to the solution, keeping the mixture at 220 °C for 3 hours, allowing the resulting material to cool down, adding water and acetic acid, filtering off deposited solids and recrystallizing from ethanol to obtain 5-hydroxy-2-(4-octyloxyphenyl)pyrimidine (29 g) (m.p.: 131.2 - 132.5 °C).

(iv) Preparation of the captioned compound

A mixture of 5-hydroxy-2-(4-octyloxyphenyl)pyrimidine (9 g), N,N-dicyclohexylcarbodiimide (11 g) and 4-dimethylaminopyridine (2 g) was dissolved in dichloromethane (100 mℓ), followed by adding heptanoic

acid (5 g) to the solution, agitating the mixture at room temperature for 3 hours, filtering off deposited solids, washing the organic layer with 2N-NaOH aqueous solution and then with water until the washing water became neutral, distilling off the solvent and recrystallizing the residue from ethanol to obtain the objective 5-heptanoyloxy(4-octyloxyphenyl)pyrimidine. This product exhibited liquid crystal phases and its phase transition points were as follows:

$$C \xrightarrow{63.4\,^{\circ}C} SC \xrightarrow{69.7\,^{\circ}C} N \xrightarrow{89.7\,^{\circ}C} I$$

Example 2

Preparation of 5-octanoyloxy-2-(4-octylphenyl)pyrimidine

The step (i) of Example 1 was repeated except that 4-hydroxybenzamidine hydrochloride used in (i) of Example 1 was replaced by 4-octylbenzamidine hydrochloride, to obtain 5-ethoxy-2-(4-octylphenyl)-pyrimidine, followed by repeating the step (iii) of Example 1 except that this pyrimidine was used in place of 5-ethoxy-2-(4-octyloxyphenyl)pyrimidine used in the step (iii) of Example 1, to obtain 5-hydroxy-(4-octylphenyl)pyrimidine. This compound and octanoic acid were subjected to dehydration-condensation in the same manner as in the step (iv) of Example 1 to obtain the objective 5-octanoyloxy-2-(octylphenyl)-pyrimidine. This product was monotropic and exhibited liquid crystal phases. Its phase transition points were as follows:

$$C \xrightarrow{79.4\,^{\circ}C} I$$
$$I \xrightarrow{74.4\,^{\circ}C} SA$$

Example 3 (Use example 1)

A liquid crystal composition a consisting only of the following compounds of the present invention was prepared:

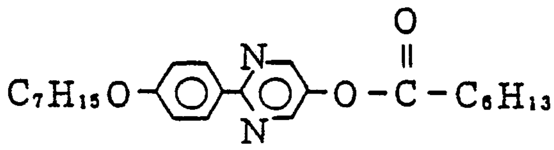 20 wt.%

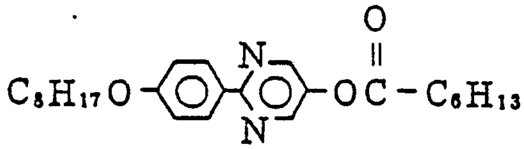 20 wt.%

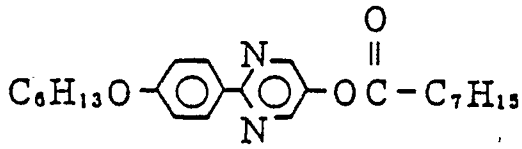 10 wt.%

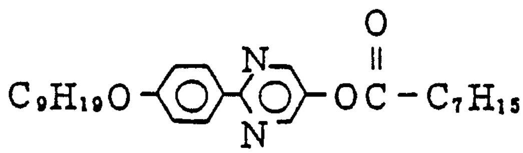 10 wt.%

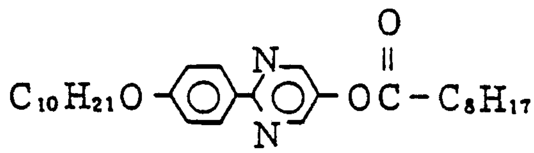 20 wt.%

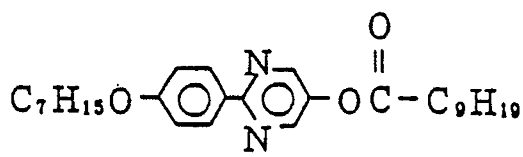 10 wt.%

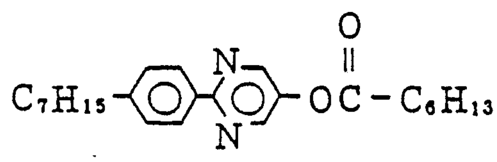 10 wt.%

The liquid crystal composition A had a m.p. of 38.0°C, formed SC phase on the higher temperature side, N phase at 73.7°C and isotropic liquid at 88.4°C.

As described above, when several compounds of the present invention alone are combined, it is possible to constitute a liquid crystal composition exhibiting SC phase within a broad temperature range including room temperature. To this liquid crystal composition A (55% by weight) were added the following optically active compounds to prepare a liquid crystal composition B:

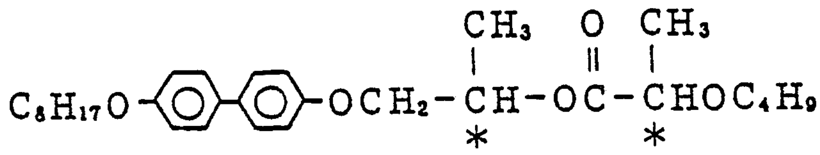

10 wt.%

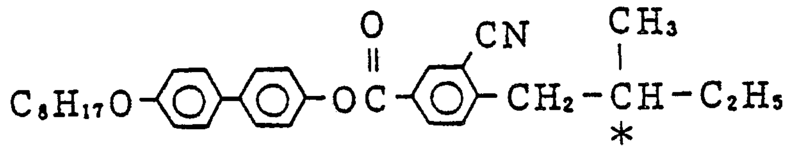

5 wt.%

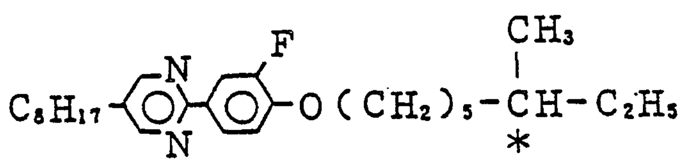

10 wt. %

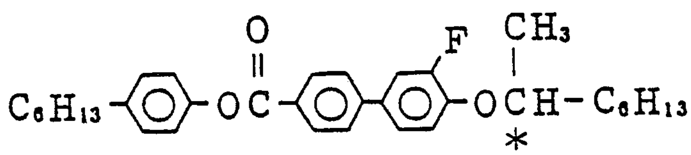

10 wt. %

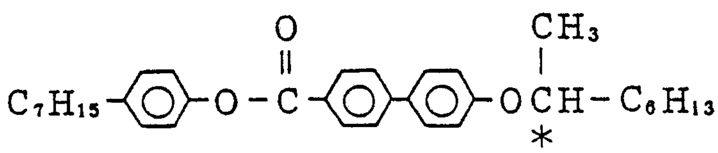

10 wt. %

This liquid crystal composition B had a m.p. of 13.0 °C, exhibited SC* phase within a higher temperature range than the m.p., and formed SA phase at 60.0 °C, N phase at 60.4 °C and isotropic liquid at 72.7 °C. Further, its supercooled state was observed down to -8.0 °C, it had SC* phase down to this temperature and no other smectic phase was observed.

In addition, its spontaneous polarization value was 31.2 nC/cm$^2$ and its tilt angle was 30.0°.

The liquid crystal composition B was filled in a cell of 2 µm thick provided with transparent electrodes, each obtained by coating PVA (polyvinyl alcohol) as an aligning agent and then rubbing the resulting surface to subject it to a parallel aligning treatment, followed by placing the resulting liquid crystal element between two crossed polarizers and impressing an electric field. As a result, change in the intensity of transmitted light was observed by impressing 15 V. The response time was sought from the change in the intensity of transmitted light to give about 108 µsec at 25 °C.

From the foregoing, it is seen that when the compound of the formula (I) of the present invention is blended with optically active liquid crystal compounds, a ferroelectric chiral smectic C liquid crystal composition having a broad region of SC* phase and superior response properties is obtained.

Example 4 (Use example 2)

To the smectic C liquid crystal composition A (50% by weight) prepared in Example 3 were added the following optically active compounds to prepare a liquid crystal composition C:

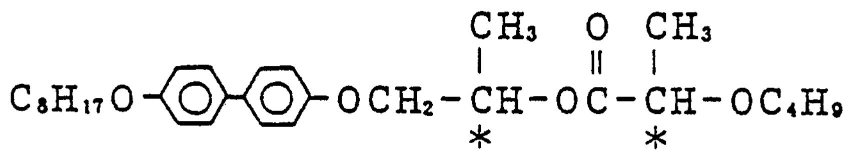

15 wt. %

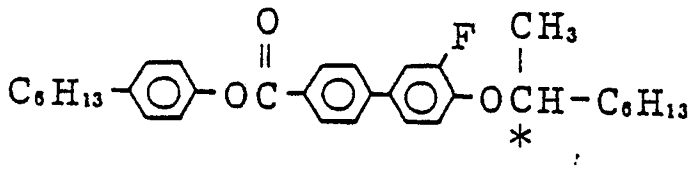

10 wt.%

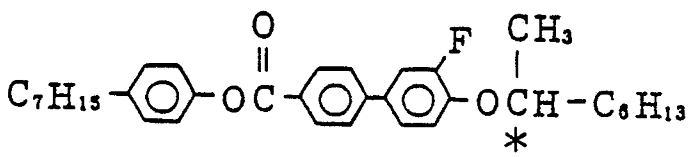

10 wt.%

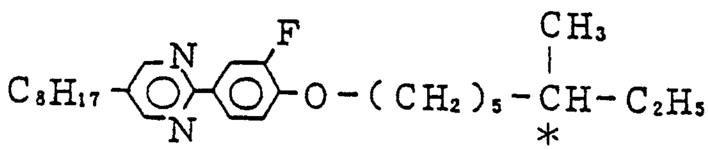

10 wt.%

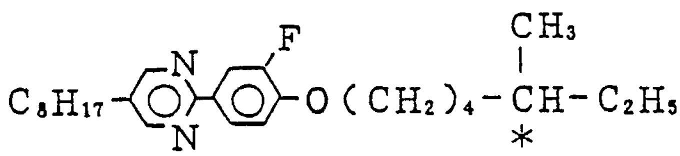

5 wt. %

This liquid crystal composition C had a m.p. of 17.0°C, exhibited SC* phase within a temperature region higher than the m.p. and formed SA phase at 56.2°C, N phase at 57.0°C and isotropic liquid at 66.0°C. Further, its supercooled state was observed down to -5.0°C, it had SC* phase down to this temperature and no other smectic phase was observed.

In addition, its spontaneous polarization value was 23.5 nC/cm$^2$ at 25°C and its tilt angle was 28.5°. With the liquid crystal composition C, the response time was sought under the same conditions as in Example 3 to give about 110 μsec at 25°C.

As seen from the foregoing, when the compound of the formula (I) of the present invention is blended with optically active liquid crystal compounds, a ferroelectric chiral smectic liquid crystal composition having properties suitable to practical uses is obtained.

## Claims

1. Compounds of the formula:

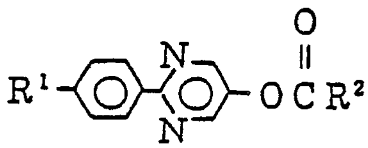

(I)

in which $R^1$ is an alkyl or alkoxy group containing up to 20 carbon atoms and $R^2$ is an alkyl group containing up to 20 carbon atoms.

2. Compound according to claim 1, in which $R^1$ is an alkyl or alkoxy group containing from 6 to 12 carbon atoms.

3. Compounds according to claim 1 or claim 2 in which $R^2$ is an alkyl group containing from 6 to 12 carbon atoms.

4. A liquid crystal composition comprising at least two compounds, at least one of which is a compound of formula (I) as claimed in claim 1.

5. A liquid crystal composition according to claim 4, which exhibits a nematic liquid crystal phase.

6. A liquid crystal composition according to claim 4, which exhibits a smectic liquid crystal phase.

7. A chiral smectic liquid crystal composition comprising a liquid crystal composition which comprises at least two components, at least one of which is a compound of formula (I) as claimed in claim 1 and exhibits a smectic liquid crystal phase and at least one optically active compound.

8. A light switching element using a chiral smectic liquid crystal composition comrprising a liquid crystal composition which comprises at least two components at least one of which is a compound of formula (I) as claimed in claim 1 and exhibits a smectic liquid crystal phase and at least one optically active compound.

European Patent Office

# EUROPEAN SEARCH REPORT

Application Number

EP 88 31 0821

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 244 129 (AJINOMOTO CO.) * Pages 6,7, examples 10-12; claims * --- | 1-8 | C 07 D 239/34<br>C 09 K 19/34 |
| P,X | EP-A-0 260 077 (AJINOMOTO CO.) * Page 5, example 2; page 9, table 1; page 10, table 2; claims * ----- | 1-8 | |

| TECHNICAL FIELDS SEARCHED (Int. Cl.4) |
|---|
| C 07 D 239/00<br>C 09 K 19/00 |

The present search report has been drawn up for all claims

| Place of search | Date of completion of the search | Examiner |
|---|---|---|
| THE HAGUE | 20-03-1989 | BESLIER L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)